Europäisches Patentamt

European Patent Office

Office européen des brevets

(19)

(11) Veröffentlichungsnummer: **0 034 338**
**B1**

(12) **EUROPÄISCHE PATENTSCHRIFT**

(45) Veröffentlichungstag der Patentschrift:
22.12.82

(51) Int. Cl.³: **B 01 J 37/02,** B 01 J 23/76,
C 07 C 29/15

(21) Anmeldenummer: 81100994.3

(22) Anmeldetag: 13.02.81

(54) Katalysator zur Synthese von Methanol und höhere Alkohole enthaltenden Alkoholgemischen.

(30) Priorität: 14.02.80 DE 3005551

(43) Veröffentlichungstag der Anmeldung:
26.08.81 Patentblatt 81/34

(45) Bekanntmachung des Hinweises auf die Patenterteilung:
22.12.82 Patentblatt 82/51

(84) Benannte Vertragsstaaten:
AT BE CH DE FR GB LI LU NL SE

(56) Entgegenhaltungen:
DE-A-1 767 881
DE-A-2 056 612
DE-A-2 154 074
DE-A-2 928 434
DE-A-2 946 137
DE-B-1 230 403
DE-C-967 944

(73) Patentinhaber: SÜD-CHEMIE AG, Lenbachplatz 6,
D-8000 München 2 (DE)

(72) Erfinder: Hofstadt, Carl-Ernst, Dr. Dipl.-Chem.,
Wehrlestrasse 29, D-8000 München 2 (DE)
Erfinder: Kochloefl, Karel, Dr. Dipl.-Ing., Kreuzstrasse 2,
D-8052 Moosburg (DE)
Erfinder: Bock, Ortwin, Klötzelmüllerstrasse 58,
D-8300 Landshut (DE)

(74) Vertreter: Patentanwälte Dipl.-Ing. Splanemann
Dipl.-Chem. Dr. B. Reitzner, Tal 13,
D-8000 München 2 (DE)

## Katalysator zur Synthese von Methanol und höhere Alkohole enthaltenden Alkoholgemischen

Die Erfindung betrifft Katalysatoren zur Synthese von Methanol und höhere Alkohole enthaltenden Alkoholgemischen.

Die Synthese von aliphatischen Alkoholen aus Synthesegasen bzw. Kohlenmonoxid und Wasserstoff ist bekannt; je nach der Art des Katalysators und der Reaktionsbedingungen wurden die einzelnen technischen Verfahren als Synol-, Oxyl- und Isobutylölsynthese bezeichnet.

Für die Synolsynthese wurden hauptsächlich alkalisierte Eisen-Schmelz-Katalysatoren verwendet, die durch Modifizierung von ursprünglich für die Fischer-Tropsch-Synthese entwickelten Katalysatoren erhalten wurden. Die Prozessbedingungen lagen bei Temperaturen von 190–200°C, Drücken von 18–25 bar und Raumgeschwindigkeiten von 100–200 Liter pro Stunde und Liter Katalysator bei einem $H_2/CO$-Verhältnis von 1 im Frischgas (DE-C-933 388). Die flüssigen Reaktionsprodukte enthielten etwa 42 Gew.-% aliphatische Alkohole mit einem Siedepunkt von 40–420°C, den Rest bildeten andere Sauerstoffverbindungen, wie Ester, Aldehyde, Ketone und Carbonsäuren, sowie Kohlenwasserstoffe.

Die Oxylsynthese (DE-C-902 851, DE-C-897 698 und 923 127) verlief bei Temperaturen von 180–220°C, Drücken von 10–30 bar, Raumgeschwindigkeiten von 100–300 Liter Synthesegas pro Stunde und Liter Katalysator, wobei das $H_2/CO$-Verhältnis im Frischgas 1,2 bis 2:1 betrug. Als Katalysatoren dienten Eisen-Fällungskatalysatoren, die mit Cer oder Vanadium promotiert waren. In den flüssigen Reaktionsprodukten fanden sich neben etwa 55 Gew.-% aliphatischen Alkoholen noch andere sauerstoffhaltige Verbindungen sowie Kohlenwasserstoffe. Es bildeten sich hauptsächlich $C_2$- bis $C_{18}$-Alkohole, wobei deren prozentualer Anteil mit steigender C-Zahl abfiel.

Ferner wurden modifizierte Eisenkatalysatoren mit Kalium, Chrom, Mangan und Barium als Aktivatoren verwendet, die zu einer Erhöhung des Anteils an sauerstoffhaltigen Verbindungen führten (DE-C 937 706, 959 911 und 967 944).

Diese beiden Synthesen haben den Nachteil, dass die Selektivität der Bildung von aliphatischen Alkoholen ziemlich niedrig ist und deren Abtrennung von den anderen Reaktionsprodukten ein kompliziertes und aufwendiges Verfahren darstellt.

Die Isobutylölsynthese wurde bevorzugt mit durch Alkali promotierten Zinkoxid- und Chromoxid-Katalysatoren bei Temperaturen von 400–450°C und Drücken von 250–350 bar durchgeführt. Neben Methanol (etwa 50 Gew.-%) überwog in den Reaktionsprodukten Isobutanol (etwa 11–14 Gew.-%), den Rest bildeten höhere aliphatische Alkohole, andere sauerstoffhaltige Verbindungen und Wasser.

Die Synol-, Oxyl- und Isobutylölsynthese wurden durch wirtschaftlichere Verfahren zur Herstellung von Alkoholen über die neuentwickelte Oxosynthese bzw. die Hydratisierung von Olefinen in den Jahren um 1950 abgelöst. Später wurden eisen-, kupfer-, bor- und kaliumhaltige Katalysatoren entwickelt (SU-Patentschriften 124 908, 243 606 und 386 899), die zur Synthese von aliphatischen Alkoholen aus CO und $H_2$ ($H_2/CO$-Verhältnis 10) bei Temperaturen von 160–225°C, Drücken von 100–300 bar und Raumgeschwindigkeiten von 2000 Liter Synthesegas pro Stunde und Liter Katalysator eingesetzt wurden. Bei einem Umsatz von 14 Mol.-% betrug der Gehalt an sauerstoffhaltigen Verbindungen etwa 60–90 Gew.-%, wobei der Anteil an primären aliphatischen Alkoholen je nach der Art der verwendeten Katalysatoren und den angewendeten Reaktionsbedingungen bis auf 80% anstieg.

Obwohl bei dieser Synthese die Ausbeute an aliphatischen Alkoholen hoch ist, weist das Verfahren ähnliche Nachteile wie die vorher beschriebenen auf. Es entstehen sauerstoffhaltige Verbindungen mit einem Siedebereich bis 450°C, in welchen $C_2$- bis $C_{18}$-Alkohole vertreten sind.

Da die Erzeugung von aliphatischen Alkoholen über die Oxosynthese oder die Hydratisierung von Olefinen unmittelbar vom Erdöl als Rohstoffgrundlage abhängt, werden angesichts der in den letzten Jahren zunehmenden Verschlechterung der Rohstoffsituation neue Wege gesucht, die zur Milderung der Abhängigkeit der petrochemischen Industrie von Erdöleinfuhren beitragen sollen.

Der Erfindung liegt die Aufgabe zugrunde, Katalysatoren zur Verfügung zu stellen, die die Synthese von Methanol und höhere Alkohole enthaltenden Alkoholgemischen aus Synthesegas bzw. Kohlenmonoxid und Wasserstoff ermöglichen, wobei die Zusammensetzung der gebildeten Alkohole hauptsächlich auf $C_1$- bis $C_4$-Alkohole beschränkt bleibt und der Anteil der einzelnen Alkohole durch die Wahl der Katalysatorzusammensetzung und der Reaktionsbedingungen steuerbar ist. Ausserdem soll mit Hilfe dieser Katalysatoren erreicht werden, dass die Kohlenmonoxidumsetzung aus Gründen der Wirtschaftlichkeit unter relativ milden Reaktionsbedingungen (Temperaturen bis 400°C und Drücke bis 150 bar) erfolgt. Es wurde gefunden, dass kupfer- und zink- sowie gegebenenfalls aluminiumhaltige Katalysatoren, die bisher nur zur Niederdruckmethanolsynthese verwendet wurden, als Ausgangskatalysatoren für eine Weiterentwicklung in der angestrebten Richtung geeignet waren.

Gegenstand der Erfindung sind somit Katalysatoren zur Synthese von Methanol und höhere Alkohole enthaltenden Alkoholgemischen auf der Grundlage von Kupfer-, Zink- und gegebenenfalls Aluminiumoxid enthaltenden Ausgangskatalysatoren, die dadurch erhältlich sind, dass man entweder (a) aus löslichen Salzen des Kupfers, Zinks und gegebenenfalls Aluminiums (Hauptbestandteile) durch gemeinsame Fällung mit Promotorverbindungen in alkalischem Medium einen Niederschlag erzeugt, der nach Entfernung der Fremdionen calciniert wird; oder (b) ein Gemisch der Oxide des Kupfers, Zinks und gegebenenfalls des Aluminiums mit Lösungen der Promotorverbindungen imprägniert und das erhaltene Produkt calciniert; wobei die Promotorverbindungen Verbindungen von Chrom, Cer, Lanthan, Mangan, Thorium oder von Kombinationen dieser Elemente darstellen, und wobei auf einer beliebigen Herstellungsstufe Alkaliverbindungen zugesetzt werden.

Die verwendeten Alkaliverbindungen brauchen bei der Alternative (a) nicht gesondert zugesetzt zu werden. Es reichen häufig die nach der Fällung im Niederschlag enthaltenen Alkalikationen des zur Fällung verwendeten alkalischen Mediums aus. Bevorzugte Alkaliverbindungen sind Kaliumverbindungen, ferner Rubidium- und Cäsium-Verbindungen. Lithium- und Natriumverbindungen werden weniger bevorzugt.

Die nach beiden Varianten erhaltenen Niederschläge bzw. Vorprodukte könnten vor der Imprägnierung mit den Promotorverbindungen bzw. den Alkaliverbindungen oder aber auch danach, d.h. unmittelbar vor dem Calcinieren, zu Formkörpern, z.B. zu Pellets, Tabletten und Strangpresslingen gepresst werden, wobei Gleitmittel, wie Graphit, zugesetzt werden können. Es können z.B. zylindrische Tabletten mit einem Durchmesser bzw. einer Höhe von 3–5 mm hergestellt werden.

Die Zusammensetzung der Ausgangskatalysatoren kann innerhalb weiter Grenzen schwanken. Eine bevorzugte Ausführungsform der Erfindung ist dadurch gekennzeichnet, dass die Ausgangskatalysatoren 18 bis 45 Gew.-%, vorzugsweise 25 bis 40 Gew.-% Kupferoxid; 24 bis 50 Gew.-%, vorzugsweise 30 bis 45 Gew.-% Zinkoxid; 5 bis 25 Gew.-%, vorzugsweise 10 bis 18 Gew.-% Aluminiumoxid und 0,03 bis 3,4 Gew.-%, vorzugsweise 1,7 bis 2,5 Gew.-% Kalium (berechnet als $K_2O$) enthalten, wobei Kupfer und Zink in einem Atomverhältnis von 0,4 bis 1,9 vorliegen.

Die Promotorverbindungen liegen gewöhnlich in Mengen von 1 bis 25 Gew.-%, vorzugsweise in Mengen von 3–18 Gew.-% (berechnet als Oxide) vor. Die Oxide können in verschiedenen Wertigkeitsstufen vorliegen.

Insbesondere können die erfindungsgemässen Katalysatoren dadurch erhalten werden, dass man entweder (a) aus einer Lösung der wasserlöslichen Salze, insbesondere der Nitrate, der Hauptbestandteile und der Promotorelemente, durch Zusatz von Alkalicarbonat, insbesondere von Kaliumcarbonat, bei 50 bis 80°C, vorzugsweise bei 60 bis 70°C, einen Niederschlag erzeugt, diesen abtrennt, wäscht und trocknet; oder (b) das durch thermische Zersetzung einer Kupfer-Zink-Ammincarbonatlösung in Gegenwart von suspendiertem Aluminiumoxid erhaltene Oxidgemisch mit Salzen, vorzugsweise Nitraten, der Promotorelemente, imprägniert; und die nach (a) bzw. (b) erhaltenen Produkte bei 350 bis 450°C, vorzugsweise bei 380 bis 400°C, calciniert.

Nach einer besonders bevorzugten Ausführungsform kann man die erfindungsgemässen Katalysatoren dadurch erhalten, dass man den Niederschlag von Variante (a) durch kontinuierliche Fällung der wasserlöslichen Hauptbestandteile und der Promotorelemente mit Alkalicarbonat erzeugt. Diese Verfahrensvariante wird vorzugsweise so durchgeführt, dass man eine wässrige Lösung der wasserlöslichen Hauptbestandteile und der Promotorelemente und eine wässrige Alkalicarbonatlösung einer Mischkammer mit einer Rührvorrichtung zuführt und die Suspension des in der Mischkammer gebildeten Niederschlages zur Alterung in ein Beruhigungsgefäss überführt.

Der für die Fällung erforderliche pH-Wert kann auf einfache Weise dadurch eingestellt werden, dass man die Zufuhrgeschwindigkeit einer der beiden wässrigen Lösungen variiert. Vorzugsweise wird der pH-Wert auf 6,8 bis 7,0 eingestellt.

Bei der Variante (a) können als wasserlösliche Salze der Hauptbestandteile und der Promotorelemente neben den Nitraten auch andere Salze verwendet werden, beispielsweise die Chloride und Sulfate. Allerdings muss in diesen Fällen das Auswaschen der Niederschläge etwas gründlicher vorgenommen werden. Ferner sind beispielsweise die Acetate gut geeignet, da Acetatreste beim Calcinieren rückstandslos entfernt werden können. Die Fällung wird gewöhnlich bis zu einem pH-Wert von 6–8, vorzugsweise von 6,8–7,2, durchgeführt. Der Niederschlag kann in üblicher Weise, beispielsweise durch Filtrieren, vom wässrigen Fällungsmedium abgetrennt werden. Das Waschen kann auf dem Filter oder durch Aufschlämmen des Niederschlags mit entionisiertem Wasser erfolgen.

Nach einer Abwandlung der Variante (b) kann das Oxidgemisch auch durch mechanische Vermischung der einzelnen Oxide der Hauptbestandteile erhalten werden. Ferner können die Oxide der Hauptbestandteile ähnlich wie bei Variante (a) gemeinsam ausgefällt werden, worauf das erhaltene Oxidgemisch mit den Salzen der Promotorelemente und gegebenenfalls mit den Alkaliverbindungen imprägniert wird.

Die Alkaliverbindung, insbesondere die Kaliumverbindung, kann auch durch eine Nachimprägnierung auf die bereits mit den Promotorverbindungen imprägnierten, gegebenenfalls vorcalcinierten, Ausgangskatalysatoren aufgebracht werden. Die so imprägnierten Katalysatoren werden einer Nachcalcinierung unterzogen. Der Kaliumgehalt (berechnet als $K_2O$) beträgt danach vorzugsweise 0,03–3,4 Gew.-%, insbesondere 1,7–2,5 Gew.-%.

Als Kaliumverbindungen verwendet man bei allen Imprägnierungen vorzugsweise Kaliumhydroxid, Kaliumcarbonat, Kaliumhydrogencarbonat, Kaliumacetat, Kaliumchromat oder -dichromat bzw. deren Gemische. Die bevorzugte Substanz ist Kaliumcarbonat. Die Verwendung von Kaliumchromat bzw. -dichromat hat den Vorteil, dass sowohl das Promotorelement Chrom als auch das Kalium in Form einer einzigen Verbindung eingeführt werden können.

Der Nachimprägnierung schliesst sich gewöhnlich eine Nachcalcinierung bei 350–450°C, vorzugsweise bei 380–400°C, an.

Der Katalysator wird üblicherweise dadurch aktiviert, dass er einer reduzierenden Nachbehandlung unterzogen wird. Diese wird vorzugsweise so durchgeführt, dass zunächst mit Hilfe eines eine kleine Menge Wasserstoff enthaltenden inerten Gases, wie Stickstoff, reduziert wird. Der Stickstoff enthält üblicherweise zunächst etwa 2,0 Vol.-% Wasserstoff. Dann wird der Wasserstoffanteil allmählich erhöht, bis zuletzt mit reinem Wasserstoff reduziert wird. Die Reduktion erfolgt im allgemeinen bei atmosphärischem Druck, wobei gleichzeitig mit der Erhöhung des Wasserstoffanteils im reduzierenden Gas die Temperatur allmählich von etwa 145, vorzugsweise von 170 auf 350°C erhöht wird. Üblicherweise wird mit einer Raumgeschwindigkeit von 1000 bis 2000 Liter Reduktiongas pro Stunde und Liter Katalysator aktiviert. Die Dauer der reduzierenden Nachbehandlung beträgt unter diesen Bedingungen im allgemeinen 16–18 Stunden.

Gegenstand der vorliegenden Erfindung ist ferner die Verwendung der vorstehend beschriebenen Katalysatoren zur Synthese von Methanol und höhere Alkohole enthaltenden Alkoholgemischen, wobei die Synthese bei einer Temperatur von 250 bis 400°C, vorzugsweise von etwa 350°C; bei einem Druck von 80 bis 150 bar, vorzugsweise bei etwa 100 bar; und bei einer Raumgeschwindigkeit von 1000 bis 10 000, vorzugsweise etwa 3000 Liter pro Stunde und Liter Katalysator mit einem Prozessgas durchgeführt wird, das 25 bis 30 Vol.-%, vorzugsweise etwa 27 Vol.-% CO; 0 bis 8 Vol.-% $N_2$; 0 bis 5 Vol.-% $CO_2$; 0 bis 5 Vol.-% $CH_4$; Rest $H_2$ enthält.

Die erhaltenen Reaktionsprodukte werden zur Kondensation der flüssigen Produkte auf 10–15°C abgekühlt, worauf die Anteile an gasförmigen und flüssigen Produkten gemessen und getrennt gaschromatographisch analysiert werden.

Es wurde festgestellt, dass bei Verwendung von Katalysatoren, die nur Kupfer und Zink bzw. Kupfer, Zink und Aluminium enthielten, lediglich 1–2 bzw. 1–4 Gew.-% höhere Alkohole neben 90–94 Gew.-% Methanol als flüssige Reaktionsprodukte erhalten wurden. Durch die Einführung von 0,03–3,4 Gew.-% Kalium in die kupfer-, zink- und aluminiumhaltigen Katalysatoren stieg der Anteil an höheren Alkoholen auf 8–15 Gew.-%, während die Ausbeute an flüssigen Reaktionsprodukten insgesamt abnahm. Ferner wurde festgestellt, dass die Anfangsaktivität derartiger Katalysatoren mit einem Kupfer-Zink-Atomverhältnis von 2–3:1 nach einer Laufzeit von 200 Stunden deutlich nachliess. Die bevorzugten erfindungsgemässen Katalysatoren mit einem Kupfer-Zink-Atomverhältnis von 0,4–1,9 brachten bessere Standzeiten.

Die Dotierung mit Chrom als Chrom(III)-oxid in einer Grössenordnung von 2–10 Gew.-% führte nicht nur zu einer Zunahme des Anteils an höheren aliphatischen Alkoholen in den flüssigen Reaktionsprodukten (20–30 Gew.-%), sondern auch zu einer weiteren Verbesserung der Standzeiten. In der Fraktion der $C_2$- bis $C_5$-Alkohole überwogen Propanole und Butanole, insbesondere Propanol-1.

Ähnliche Wirkungen wie Chrom zeigten Cer und Lanthan als Promotoren. Beide Elemente führten zu höheren Ausbeuten an flüssigen Reaktionsprodukten, wobei die Bildung von Isobutanol bevorzugt wurde.

Die kombinierte Promotierung mit Chrom und Thorium (5–10% $Cr_2O_3$, 5–10% $ThO_2$) ergab nicht nur höhere Ausbeuten an flüssigen Produkten, sondern auch grössere Mengen an Propanolen und Butanolen, insbesondere von Isobutanol.

Die Wirkung von Chrom und Mangan in einer Kombination (3–5% $Cr_2O_3$, 5–10% MnO) zeigte sich durch eine stark bevorzugte Bildung von Äthanol und Propanolen, insbesondere von Äthanol.

Die mit den erfindungsgemässen Katalysatoren erzielten Ergebnisse zeigen, dass durch eine gezielte Promotierung (Chrom-Mangan oder Chrom-Thorium) bei der Synthese ein Alkoholgemisch gewonnen werden kann, das neben Methanol höhere aliphatische Alkohole, und zwar entweder bevorzugt Äthanol und Propanol-1 oder Isobutanol (2-Methylpropanol-1) und Propanol-1 enthält.

Vorteilhaft ist ausserdem, dass bei der Anwendung dieser Katalysatoren hauptsächlich $C_1$- bis $C_6$-Alkohole gebildet werden und die Menge an unerwünschten Kohlenwasserstoffen unter 5% liegt.

Ein Alkoholgemisch, bestehend aus Methanol und höheren aliphatischen Alkoholen, insbesondere mit Propanolen und Butanolen, kann als Treibstoff allein oder im Verschnitt mit Benzin zum Betrieb von Otto-Motoren eingesetzt werden. Wird ein solches Gemisch mit Benzin vermischt, so erfolgt aufgrund der Anwesenheit der höheren Alkohole keine Phasentrennung, wenn zufällig Wasser in kleiner Menge in das Benzin kommt.

Darüberhinaus stellt das mit Hilfe der erfindungsgemässen Katalysatoren erhaltene Alkoholgemisch eine Rohstoffquelle für die chemische Industrie dar. Nach der destillativen Abtrennung des Methanols und weiterer Fraktionierung der höheren aliphatischen Alkohole können zusätzliche Fraktionen gewonnen werden, wobei die $C_2$- und $C_3$-Alkohole zur Herstellung von Äthylen und Propylen und die $C_4$-Alkohole zur Erzeugung von Lösungsmitteln und Weichmachern verwendet werden können. Falls der Schwerpunkt der Verarbeitung der höheren aliphatischen Alkohole bei der Gewinnung von Olefinen liegt, empfiehlt sich insbesondere der Einsatz von chrom- und mangandotierten Katalysatoren.

Die Erfindung ist durch die nachstehenden Beispiele in nicht einschränkender Weise erläutert.

Beispiel 1

In 9000 ml einer Kupferammincarbonatlösung mit einem Gehalt von 4,11 g Cu/100 ml, 8,8 g $NH_3$/100 ml und 7,4 g $CO_2$/100 ml wurden 355 g Zinkoxid unter Rühren gelöst und 177 g Al₂O₃ zugegeben. Unter fortwährendem Rühren und Ersatz des verdampfenden Wassers wurde die Suspension durch Kochen thermisch zersetzt, bis eine entnommene Probe ein farbloses Filtrat ergab. Der Niederschlag wurde abfiltriert, und der erhaltene Filterkuchen wurde in dünner Schicht 4 Stunden bei 400°C calciniert. Das erhaltene Produkt wurde mit 2% seines Gewichts an Naturgraphit vermischt und zu zylindrischen Tabletten mit einem Durchmesser von 3 mm und einer Höhe von 3 mm verpresst.

150 g der so erhaltenen Tabletten wurden bei Raumtemperatur 20 Minuten in einer wässrigen Lösung von 96,5 g $K_2CO_3$ in 200 ml Wasser getaucht und anschliessend 2 Stunden bei 120°C getrocknet und 3 Stunden bei 400°C calciniert. Die chemische Zusammensetzung und die BET-Oberfläche des mit O bezeichneten Katalysators ist in Tabelle I angegeben.

In einem mit einem flüssigen Medium beheizten Rohrreaktor (Rohrdurchmesser 18 mm, Rohrlänge 1000 mm) wurden 30 ml des vorstehend beschriebenen Katalysators O mit einem Gas, bestehend aus 1,2 Vol.-% $H_2$, Rest $N_2$, über 40 Stunden bei 145–450°C aktiviert. Der Temperaturanstieg zwischen 145–250°C betrug etwa 2°C/Stunde; nach Erreichen von 250°C wurde der zu prüfende Katalysator mit reinem Wasserstoff 5 Stunden behandelt, und die Temperatur wurde während dieser Zeit auf 350°C erhöht. Anschliessend wurde Synthesegas mit einer Zusammensetzung von

CO 29,5 Vol.-%
$CO_2$ 1,5 Vol.-%
$CH_4$ 1,4 Vol.-%
$N_2$ 5,0 Vol.-%
$H_2$ Rest

dem Reaktor zugeführt, wobei ein Druck von 100 bar und eine Raumgeschwindigkeit von 2600 Liter Gas pro Stunde und Liter Katalysator eingestellt wurden. Die Ergebnisse dieses Versuchs und die Zusammensetzung der Reaktionsprodukte sind in Tabellen II und III zusammengefasst.

Beispiel 2

Die thermische Zersetzung einer Kupferammincarbonatlösung sowie die Filtration und Calcinierung erfolgte wie im Beispiel 1 beschrieben. Das erhaltene Produkt wurde mit einer Lösung von 190 g $Cr(NO_3)_3 \cdot 9\ H_2O$ in 200 ml entionisiertem Wasser versetzt und anschliessend 3 Stunden bei 400°C calciniert. Die Oxide wurden nun mit 2% ihres Gewichts an Naturgraphit vermischt und zu zylindrischen Tabletten mit einem Durchmesser von 3 mm und einer Höhe von 3 mm verpresst.

150 g der so erhaltenen Tabletten wurden wie im Beispiel 1 angegeben in $K_2CO_3$-Lösung getaucht, getrocknet und calciniert.

Die chemische Zusammensetzung und die BET-Oberfläche des mit 1 bezeichneten Katalysators sind in Tabelle I angegeben. Die Aktivierung des Katalysators 1 und die Reaktion mit Synthesegas wurde wie in Beispiel 1 durchgeführt. Die Ergebnisse dieses Versuches und die Zusammensetzung der Reaktionsprodukte sind in Tabelle II und III zusammengefasst.

Beispiel 3

Die thermische Zersetzung einer Kupferammincarbonatlösung sowie die Filtration und Calcinierung erfolgte wie im Beispiel 1 beschrieben. Das erhaltene Produkt wurde, wie in Beispiel 2 angegeben, weiterverarbeitet; anstelle von Chrom(III)-nitrat-Lösung wurde jedoch eine wässrige Lösung von 97,4 g Cer(III)-nitrat-hexahydrat in 200 ml Wasser zugegeben. 150 g der so erhaltenen Tabletten wurden, wie im Beispiel 1 angegeben, in $K_2CO_3$-Lösung getaucht, getrocknet und calciniert.

Die chemische Zusammensetzung und die BET-Oberfläche des mit 2 bezeichneten Katalysators sind in Tabelle I angegeben. Die Aktivierung des Katalysators 2 mit Wasserstoff und die Reaktion mit Synthesegas wurde wie in Beispiel 1 durchgeführt. Die Ergebnisse dieses Versuchs und die Zusammensetzung der Reaktionsprodukte sind in Tabelle II und III zusammengefasst.

Beispiel 4

Die thermische Zersetzung einer Kupferammincarbonatlösung sowie die Filtration und Calcinierung erfolgten wie in Beispiel 1 beschrieben. Das erhaltene Produkt wurde, wie in Beispiel 2 angegeben, weiterverarbeitet; anstelle von Chrom(III)-nitrat-Lösung wurde jedoch eine wässrige Lösung von 97,8 g Lanthannitrat-hexahydrat zugegeben. 150 g der so erhaltenen Tabletten wurden, wie in Beispiel 1 angegeben, in $K_2CO_3$-Lösung getaucht, getrocknet und calciniert.

Die chemische Zusammensetzung und die BET-Oberfläche des mit 3 bezeichneten Katalysators sind in Tabelle I angegeben. Die Aktivierung des Katalysators 3 mit Wasserstoff und die Reaktion mit Synthesegas wurden wie nach Beispiel 1 durchgeführt. Die Ergebnisse dieses Versuchs und die Zusammensetzung der Reaktionsprodukte sind in Tabelle II und III zusammengefasst.

Beispiel 5

Die thermische Zersetzung einer Kupferammincarbonatlösung sowie die Filtration und Calcinierung erfolgten wie im Beispiel 1 beschrieben. Das erhaltene Produkt wurde, wie in Beispiel 1 angegeben, weiterverarbeitet.

150 g der so erhaltenen Tabletten wurden 20 Minuten bei Raumtemperatur in eine wässrige Lösung von 209,5 g $K_2Cr_2O_7$ in 200 ml Wasser getaucht und anschliessend 2 Stunden bei 120°C getrocknet und 3 Stunden bei 400°C calciniert. Die chemische Zusammensetzung und die BET-Oberfläche des mit 4 bezeichneten Katalysators sind in Tabelle I angegeben.

Die Aktivierung des Katalysators 4 mit Wasserstoff und die Reaktion mit Synthesegas wurden wie nach Beispiel 1 durchgeführt. Die Ergebnisse dieses Versuchs und die Zusammensetzung der Reaktionsprodukte sind in Tabelle II und III zusammengefasst.

Beispiel 6

a) 600 g K₂CO₃ wurden in 2 Liter entionisiertem Wasser gelöst und auf 60–80°C erwärmt. 285,2 g Cu(NO₃)₂·3H₂O, 188,2 g Zn(NO₃)₂, 131,6 g Cr(NO₃)₃·9H₂O, 52,3 g Th(NO₃)₄·4H₂O und 276 g Al(NO₃)₃·9H₂O wurden in 2 Liter entionisiertem Wasser gelöst und in die vorgelegte K₂CO₃-Lösung unter Rühren langsam zugegeben; die Temperatur wurde be 60–80°C gehalten. Nach dem Versetzen mit der Nitratlösung wurde durch Zugabe einer kleinen Menge wässriger K₂CO₃-Lösung ein pH-Wert von 6,8–7,0 eingestellt, worauf noch 30 Minuten gerührt wurde. Danach wurde der Niederschlag abfiltriert und der Filterkuchen durch mehrmaliges Aufschlämmen mit je 2 Liter entionisiertem Wasser nitratfrei gewaschen (Ringprobe). Der ausgewaschene Filterkuchen wurde bei 120°C 15 Stunden getrocknet und bei 400°C 3 Stunden calciniert. Das calcinierte Produkt wurde mit 2% seines Gewichts an Naturgraphit vermischt und zu zylindrischen Tabletten mit 3 mm Durchmesser und 3 mm Höhe verpresst. Auf 150 g der so erhaltenen Tabletten wurden 8,4 g K₂CO₃, gelöst in 30 ml H₂O, aufgesprüht; anschliessend wurde 2 Stunden bei 120°C getrocknet und 3 Stunden bei 400°C calciniert.

Die chemische Zusammensetzung und die BET-Oberfläche des mit 5 bezeichneten Katalysators sind in Tabelle I angegeben.

Die Aktivierung des Katalysators 5 mit Wasserstoff und die Reaktion mit Synthesegas wurden wie in Beispiel 1 durchgeführt. Die Ergebnisse dieses Versuches und die Zusammensetzung der Reaktionsprodukte sind in Tabelle II und III zusammengefasst.

b) Nach einer weiteren Variante wurde eine kontinuierliche Fällung durchgeführt, indem eine wässrige Lösung der wasserlöslichen Hauptbestandteile und Promotorelemente und eine wässrige Alkalicarbonatlösung mit der in Variante a) angegebenen Zusammensetzung mit gleichen Geschwindigkeiten (etwa 20 ml/min) einer Mischkammer mit einem Volumen von etwa 20 ml zugeführt wurde. In der Mischkammer befand sich ein hochtouriger Rührer, ein Thermometer und eine Elektrode zur Messung des pH-Wertes. Bei der Fällung wurde die Temperatur zwischen 60 und 80°C und der pH-Wert zwischen 6,8 und 7,0 gehalten. Die Einstellung des pH-Wertes erfolgte durch Veränderung der Zugabegeschwindigkeit der Kaliumcarbonatlösung. Die Suspension des in der Mischkammer ausgefällten Niederschlages wurde aus der Mischkammer in ein Beruhigungsgefäss eingeführt, wo er nach Beendigung der Fällung noch 30 min. gealtert wurde. Die weitere Verarbeitung des gealterten Katalysator-Niederschlages erfolgte wie nach Variante a).

---

Beispiel 7

a) 560 g K₂CO₃ wurden in 2 Liter entionisiertem Wasser gelöst und mit einer Lösung von 285,2 g Cu(NO₃)₂·3H₂O, 217,6 g Zn(NO₃)₂, 101,2 g Mn(NO₃)₂·6H₂O, 65,8 g Cr(NO₃)₃·9H₂O und 276 g Al(NO₃)₃·9H₂O in 2 Liter entionisiertem Wasser, wie im Beispiel 5 beschrieben, gefällt und weiterverarbeitet.

Auf 150 g der erhaltenen Tabletten wurde 8,4 g K₂CO₃, gelöst in 30 ml Wasser, aufgesprüht; anschliessend wurde 2 Stunden bei 120°C getrocknet und 3 Stunden bei 400°C calciniert.

Die chemische Zusammensetzung und die BET-Oberfläche des mit 6 bezeichneten Katalysators sind in Tabelle I angegeben.

Die Aktivierung des Katalysators 6 mit Wasserstoff und die Reaktion mit Synthesegas wurden wie in Beispiel 1 durchgeführt. Die Ergebnisse dieses Versuches und die Zusammensetzung der Reaktionsprodukte sind in Tabelle II und III zusammengefasst.

b) Nach der kontinuierlichen Arbeitsweise von Beispiel 6, Variante b) wurde ein Katalysator unter Verwendung der vorstehend angegebenen Einsatzmengen gefällt. Die Weiterverarbeitung des Katalysators erfolgte wie nach Variante a).

Tabelle I
Zusammenstellung der promotierten kupfer-, zink-, aluminium- und kaliumhaltigen Katalysatoren (Gew.-%)

| Bezeichnung | Cu/ (CuO) | ZnO | Al₂O₃ | K | Cr₂O₃ | MnO | ThO₂ | Ce₂O₃ | La₂O₃ | GV* (600°C) | BET-Oberfläche (m²/g) |
|---|---|---|---|---|---|---|---|---|---|---|---|
| 0 | 35.2 | 33,8 | 16.9 | 3.1 | – | – | – | – | – | 8.7 | |
| | (40.5) | 31.0 | 15.5 | 2.8 | – | – | – | – | – | 8.0 | 48.4 |
| 1 | 34.2 | 32.9 | 16.4 | 3.0 | 3.3 | – | – | – | – | 7.9 | |
| | (39.4) | 30.3 | 15.1 | 2.8 | 3.0 | – | – | – | – | 7.3 | 45.4 |
| 2 | 34.3 | 32.9 | 16.4 | 3.0 | – | – | – | 3.4 | – | 7.7 | |
| | (39.5) | 30.3 | 15.1 | 2.8 | – | – | – | 3.1 | – | 7.1 | 44.5 |
| 3 | 34.4 | 32.9 | 16.4 | 3.0 | – | – | – | – | 3.4 | 7.6 | |
| | (39.6) | 30.3 | 15.1 | 2.8 | – | – | – | – | 3.1 | 7.0 | 44.1 |
| 4 | 32.9 | 31.7 | 15.7 | 2.9 | 8.9 | – | – | – | – | 7.3 | |
| | (38.1) | 29.3 | 14.5 | 2.7 | 8.2 | – | – | – | – | 6.7 | 43.2 |
| 5 | 27.3 | 29.2 | 13.7 | 3.0 | 9.2 | – | 9.2 | – | – | 6.1 | |
| | (32.0) | 27.3 | 12.8 | 2.8 | 8.6 | – | 8.6 | – | – | 5.7 | 38.3 |

Tabelle I (Fortsetzung)
Zusammenstellung der promotierten kupfer-, zink-, aluminium- und kaliumhaltigen Katalysatoren (Gew.-%)

| Bezeich-nung | Cu/ (CuO) | ZnO | $Al_2O_3$ | K | $Cr_2O_3$ | MnO | $ThO_2$ | $Cc_2O_3$ | $La_2O_3$ | GV* (600°C) | BET-Oberfläche (m²/g) |
|---|---|---|---|---|---|---|---|---|---|---|---|
| 6 | 27.3 | 33.8 | 13.7 | 3.1 | 4.5 | 9.2 | – | – | – | 6.1 | |
| | (32.0) | 31.6 | 12.8 | 2.9 | 4.2 | 8.6 | – | – | – | 5.7 | 60.9 |

* Glühverlust, hauptsächlich bedingt durch Kohlenstoff

Tabelle II
Umsatz von CO und Selektivitäten der Bildung von einzelnen Reaktionsprodukten an verschiedenen promotierten kupfer-, zink-, aluminium- und kaliumhaltigen Katalysatoren bei 350°C, 100 bar Druck und einer Raumgeschwindigkeit von 2600 Liter Synthesegas pro Std. und Liter Kat.

| Katalysator | CO-Umsatz (Mol %) | Selektivität (%) der Bildung von | | | |
|---|---|---|---|---|---|
| | | Methanol | Höheren aliph. Alkohole | $CO_2$ | Nebenprodukte (Gas u. flüss. Kohlenwasserstoffe) |
| 0 | 21.0 | 55.7 | 22.8 | 17.4 | 4.1 |
| 1 | 22.5 | 40.1 | 32.4 | 22.2 | 5.3 |
| 2 | 23.8 | 40.0 | 30.6 | 22.6 | 6.8 |
| 3 | 24.2 | 40.8 | 30.0 | 23.2 | 6.0 |
| 4 | 27.4 | 39.3 | 29.8 | 24.5 | 6.4 |
| 5 | 27.6 | 40.3 | 29.1 | 23.0 | 7.6 |
| 6 | 28.8 | 40.5 | 26.2 | 24.9 | 8.4 |

Tabelle III
Ausbeute (g pro Std. und Liter Kat. und m³ Synthesegas) an flüssigen Reaktionsprodukten und ihre Zusammensetzung an verschiedenen promotierten kupfer-, zink-, aluminium- und kaliumhaltigen Katalysatoren bei 350°C, 100 bar Druck und einer Raumgeschwindigkeit von 2600 Liter Synthesegas pro Std. und Liter Kat.

| Katalysator | Ausbeute (g/h $l_{Kat}$ m³ S.G.) an flüssigen Reaktionsprodukten | Zusammensetzung der flüssigen Anteile | | | | | | | |
|---|---|---|---|---|---|---|---|---|---|
| | | Methanol g | % | Höhere Alkohole g | % | $H_2O$ g | % | Nebenprodukte g | % |
| 0 | 52.8 | 39.0 | 73.8 | 10.1 | 19.1 | 2.7 | 5.2 | 1.0 | 1.9 |
| 1 | 53.7 | 33.6 | 62.6 | 16.1 | 30.0 | 2.7 | 5.0 | 1.3 | 2.4 |
| 2 | 54.0 | 33.9 | 62.8 | 15.9 | 29.5 | 2.8 | 5.1 | 1.4 | 2.6 |
| 3 | 55.2 | 35.1 | 63.6 | 15.4 | 27.9 | 3.1 | 5.6 | 1.6 | 2.9 |
| 4 | 63.3 | 41.1 | 65.0 | 18.7 | 29.5 | 1.2 | 1.9 | 2.3 | 3.6 |
| 5 | 62.7 | 40.4 | 64.4 | 17.8 | 28.4 | 1.5 | 2.4 | 3.0 | 4.8 |
| 6 | 62.0 | 41.8 | 67.4 | 17.3 | 28.0 | 0.4 | 0.6 | 2.5 | 4.0 |

Tabelle IV
Zusammensetzung von höheren Alkoholen, gewonnen an verschiedenen promotierten kupfer-, zink-, aluminium- und kaliumhaltigen Katalysatoren

| Katalysator | Zusammensetzung (Gew.-%) | | | | | | | |
|---|---|---|---|---|---|---|---|---|
| | $C_2H_5OH$ | $C_3H_7OH$ 2- | 1- | $C_4H_9OH$ 2- | 1- | 1- | $C_5H_{11}OH$ | Alkohole höhere als $C_5H_{11}OH$ |
| 0 | 18.1 | 2.0 | 36.1 | 2.0 | 33.4 | 1.9 | 4.0 | 2.5 |
| 1 | 14.7 | 2.1 | 41.4 | 2.6 | 31.7 | 1.7 | 3.3 | 2.5 |
| 2 | 13.8 | 2.0 | 40.3 | 2.7 | 33.2 | 1.8 | 3.8 | 2.4 |
| 3 | 13.0 | 2.0 | 39.8 | 2.8 | 33.7 | 1.7 | 4.0 | 3.0 |
| 4 | 18.3 | 2.2 | 32.5 | 1.6 | 36.8 | 1.5 | 4.3 | 2.8 |
| 5 | 11.9 | 2.2 | 26.9 | 4.0 | 42.9 | 4.3 | 5.6 | 2.2 |
| 6 | 38.2 | 5.3 | 36.1 | 2.6 | 9.5 | 2.7 | 2.7 | 2.9 |

## Patentansprüche

1. Katalysator zur Synthese von Methanol und höhere Alkohole enthaltenden Alkoholgemischen auf der Grundlage von Kupfer-, Zink- und gegebenenfalls Aluminiumoxid enthaltenden Ausgangskatalysatoren, dadurch erhältlich, dass man entweder
(a) aus löslichen Salzen des Kupfers, Zinks und gegebenenfalls Aluminiums (Hauptbestandteile) durch gemeinsame Fällung mit Promotorverbindungen in alkalischem Medium einen Niederschlag erzeugt, der nach Entfernung der Fremdionen calciniert wird; oder
(b) ein Gemisch der Oxide des Kupfers, Zinks und gegebenenfalls des Aluminiums mit Lösungen der Promotorverbindungen imprägniert und das erhaltene Produkt calciniert; wobei die Promotorverbindungen Verbindungen von Chrom, Cer, Lanthan, Mangan, Thorium oder von Kombinationen dieser Elemente darstellen, und wobei auf einer beliebigen Herstellungsstufe Alkaliverbindungen zugesetzt werden.

2. Katalysator nach Anspruch 1, dadurch gekennzeichnet, dass die Ausgangskatalysatoren 18 bis 45 Gew.-%, vorzugsweise 25 bis 40 Gew.-% Kupferoxid; 24 bis 50 Gew.-%, vorzugsweise 30 bis 45 Gew.-% Zinkoxid; 5 bis 25 Gew.-%, vorzugsweise 10 bis 18 Gew.-% Aluminiumoxid und 0,03 bis 3,4 Gew.-%, vorzugsweise 1,7 bis 2,5 Gew.-% Kalium (berechnet als $K_2O$) enthalten, wobei Kupfer und Zink in einem Atomverhältnis von 0,4 bis 1,9 vorliegen.

3. Katalysator nach Anspruch 1 oder 2, dadurch gekennzeichnet, dass die Promotorverbindungen in Mengen von 1 bis 25 Gew.-%, vorzugsweise von 3 bis 18 Gew.-% (berechnet als Oxide) vorliegen.

4. Katalysator nach einem der Ansprüche 1 bis 3, dadurch erhältlich, dass man entweder
(a) aus einer Lösung der wasserlöslichen Salze, insbesondere der Nitrate, der Hauptbestandteile und der Promotorelemente, durch Zusatz von Alkalicarbonat, insbesondere von Kaliumcarbonat, bei 50 bis 80°C, vorzugsweise bei 60 bis 70°C, einen Niederschlag erzeugt, diesen abtrennt, wäscht und trocknet; oder
(b) das durch thermische Zersetzung einer Kupfer-Zink-Ammincarbonatlösung in Gegenwart von suspendiertem Aluminiumoxid erhaltene Oxidgemisch mit Salzen, vorzugsweise Nitraten, der Promotorelemente, imprägniert; und die nach (a) bzw. (b) erhaltenen Produkte bei 350 bis 450°C, vorzugsweise bei 380 bis 400°C, calciniert.

5. Katalysator nach Anspruch 4, dadurch gekennzeichnet, dass man den Niederschlag von Variante (a) durch kontinuierliche Fällung der wasserlöslichen Hauptbestandteile und der Promotorelemente mit Alkalicarbonat erzeugt.

6. Katalysator nach Anspruch 5, dadurch gekennzeichnet, dass man eine wässrige Lösung der wasserlöslichen Hauptbestandteile und der Promotorelemente und eine wässrige Alkalicarbonatlösung einer Mischkammer mit einer Rührvorrichtung zuführt und die Suspension des in der Mischkammer gebildeten Niederschlages zur Alterung in ein Beruhigungsgefäss überführt.

7. Katalysator nach einem der Ansprüche 1 bis 6, dadurch gekennzeichnet, dass die mit den Promotorverbindungen imprägnierten Ausgangskatalysatoren einer Nachimprägnierung mit einer Kaliumverbindung und einer Nachcalcinierung unterzogen worden sind, wobei der Kaliumgehalt (berechnet als $K_2O$) 0,03 bis 3,4 Gew.-%, vorzugsweise 1,7 bis 2,5 Gew.-%, beträgt.

8. Katalysator nach Anspruch 7, dadurch gekennzeichnet, dass als Kaliumverbindungen Kaliumhydroxid, Kaliumcarbonat, Kaliumhydrogencarbonat, Kaliumacetat, Kaliumchromat oder -dichromat bzw. deren Gemische verwendet werden und die Nachcalcinierung bei 350 bis 450°C, vorzugsweise bei 380 bis 400°C durchgeführt wird.

9. Katalysator nach einem der Ansprüche 1 bis 8, dadurch gekennzeichnet, dass er zur Aktivierung einer reduzierenden Nachbehandlung unterzogen wird.

10. Katalysator nach Anspruch 9, dadurch gekennzeichnet, dass man die reduzierende Nachbehandlung zunächst mit Hilfe eines eine kleine Menge Wasserstoff enthaltenden inerten Gases, wie Stickstoff, durchführt und den Wasserstoffanteil allmählich erhöht, wobei gleichzeitig die Temperatur allmählich von 170 auf 350°C erhöht wird.

11. Verwendung des Katalysators nach einem der Ansprüche 1 bis 10 zur Synthese von Methanol und höhere Alkohole enthaltenden Alkoholgemischen.

12. Verwendung nach Anspruch 11, dadurch gekennzeichnet, dass die Synthese bei einer Temperatur von 250 bis 400°C, vorzugsweise von 350°C; bei einem Druck von 80 bis 150 bar, vorzugsweise bei 100 bar; und bei einer Raumgeschwindigkeit von 1000 bis 10 000, vorzugsweise 3000 Liter pro Stunde und Liter Katalysator mit einem Prozessgas durchgeführt wird, das 25 bis 30 Vol.-%, vorzugsweise 27 Vol.-% CO; 0 bis 8 Vol.-% $N_2$; 0 bis 5 Vol.-% $CO_2$; 0 bis 5 Vol.-% $CH_4$; Rest $H_2$ enthält.

## Claims

1. Catalyst for the synthesis of alcohol mixtures containing methanol and higher alcohols on the basis of starting catalysts containing copper oxide, zinc oxide and, optionally, aluminium oxide, obtainable either by
(a) producing from soluble salts of copper, zinc and, optionally, aluminium (principal constituents) by joint precipitation with promoter compounds in an alkaline medium a precipitate which is calcined after removal of the impurity ions; or by
(b) impregnating a mixture of the oxides of copper, zinc and, optionally, aluminium, with solutions of the promoter compounds and calcining the resultant product; wherein the promoter compounds represent compounds of chromium, cerium, lanthanum, manganese, thorium or of combinations of these elements, and wherein at any desired stage of production alkali compounds are added.

2. Catalyst according to Claim 1, characterised in that the starting catalysts contain 18 to 45% by weight, preferably 25 to 40% by weight, of copper oxide, 24 to 50% by weight, preferably 30 to 45% by weight of zinc oxide; 5 to 25% by weight, preferably 10 to 18% by weight, of aluminium oxide and 0.03 to 3.4% by weight, preferably 1.7 to 2.5% by weight, of potassium (calculated as $K_2O$), copper and zinc being present in an atomic ratio of 0.4 to 1.9.

3. Catalyst according to Claim 1 or Claim 2, characterised in that the promoter compounds are present in quantities of 1 to 25% by weight, preferably 3 to 18% by weight (calculated as oxides).

4. Catalyst according to one of Claims 1 to 3, obtainable either by
(a) producing a precipitate from a solution of the water-soluble salts, in particular the nitrates, of the principal constituents and of the promoter elements, by addition of alkali carbonate, in particular potassium carbonate, at 50 to 80°C, preferably at 60 to 70°C, separating this precipitate, washing and drying it; or by
(b) impregnating with salts, preferably nitrates, of the promoter elements the oxide mixture obtained by thermal decomposition of a copper-zinc-ammine carbonate solution in the presence of suspended aluminium oxide; and calcining the products obtained in accordance with (a) or (b) at 350 to 450%C, preferably at 380 to 400°C.

5. Catalyst according to Claim 4, characterised in that the precipitate of variant (a) is produced by continuous precipitation of the water-soluble principal constituents and the promoter elements with alkali carbonate.

6. Catalyst according to Claim 5, characterised in that an aqueous solution of the water-soluble principal constituents and of the promoter elements and an aqueous alkali carbonate solution are fed to a mixing chamber with an agitating device and the suspension of the precipitate formed in the mixing chamber is transferred into a stabilizing vessel for ageing.

7. Catalyst according to one of Claims 1 to 6, characterised in that the starting catalysts impregnated with the promoter compounds have been subjected to a reimpregnation with a potassium compound and to recalcining, the potassium content (calculated as $K_2O$) amounting to 0.03 to 3.4% by weight, preferably 1.7 to 2.5% by weight.

8. Catalyst according to Claim 7, characterised in that potassium hydroxide, potassium carbonate, potassium hydrocarbonate, potassium acetate, potassium chromate or dichromate or mixtures thereof are used as potassium compounds and the recalcining is carried out at 350 to 450°C, preferably at 380 to 400°C.

9. Catalyst according to one of Claims 1 to 8, characterised in that for activation it is subjected to a reducing aftertreatment.

10. Catalyst according to Claim 9, characterised in that the reducing aftertreatment is carried out initially by means of an inert gas, such as nitrogen, containing a small amount of hydrogen, and the proportion of hydrogen is gradually increased, the temperature being gradually increased at the same time from 170 to 350°C.

11. Use of the catalyst according to one of Claims 1 to 10 for the synthesis of alcohol mixtures containing methanol and higher alcohols.

12. Use according to Claim 11, characterised in that the synthesis is carried out at a temperature of 250 to 400°C, preferably of 350°C, at a pressure of 80 to 150 bars, preferably at 100 bars, and at a space velocity of 1000 to 10000, preferably 3000 litres per hour and litre catalyst, with a process gas which contains 25 to 30% by volume preferably 27% by volume, of CO, 0 to 8% by volume of $N_2$, 0 to 5% by volume of $CO_2$, 0 to 5% by volume of $CH_4$ and the remainder $H_2$.

**Revendications**

1. Catalyseur pour la synthèse de mélanges d'alcools contenant du méthanol et des alcools de classes supérieures, à base de catalyseurs de départ contenant des oxydes de cuivre, de zinc et le cas échéant d'aluminium, caractérisé par le fait qu'il peut être obtenu en suivant l'une des procédures ci-dessous:
a) à partir de sels solubles de cuivre, de zinc et le cas échéant d'aluminium, par précipitation chimique simultanée avec des composés promoteurs, en milieu alcalin, on produit un précipité qui, après élimination des ions étrangers est traité par calcination; ou bien
b) on imprègne avec des solutions de composés promoteurs, un mélange d'oxydes de cuivre, de zinc et le cas échéant d'aluminium et on traite le produit obtenu par calcination;
les composés promoteurs étant constitués par des composés du chrome, du cérium, du lanthane, du manganèse, du thorium ou de combinaisons de ces éléments et des composants alcalins étant ajoutés à un stade quelconque du processus de fabrication.

2. Catalyseur selon la revendication 1, caractérisé par le fait que les catalyseurs de départ contiennent 18 à 45 pour cent, de préférence 25 à 40 pour cent en poids d'oxyde de cuivre; 24 à 50 pour cent, de préférence 30 à 45 pour cent en poids d'oxyde de zinc; 5 à 25 pour cent, de préférence 10 à 18 pour cent d'oxyde d'aluminium et de 0,03 à 3,4 pour cent, de préférence 1,7 à 2,5 pour cent en poids de potassium (représenté par son poids de $K_2O$), le cuivre et le zinc étant présents dans un rapport atomique de 0,4 à 1,9.

3. Catalyseur selon la revendication 1 où 2, caractérisé par le fait que les composés promoteurs sont présents avec des quantités de 1 à 25 pour cent, de préférence de 3 à 18 pour cent en poids (représentés par leurs poids sous forme d'oxydes).

4. Catalyseur selon l'une des revendications 1 à 3, caractérisé par le fait qu'il peut être obtenu en suivant l'une des procédures ci-dessous:

a) à partir d'une solution de sels solubles, de nitrates en particulier, des éléments constitutifs principaux et des éléments promoteurs, en ajoutant du carbonate alcalin et plus particulièrement du carbonate de potassium, à une température de 50 à 80°C, de préférence de 60 à 70°C on obtient un précipité, on sépare celui-ci, on le lave et on le dessèche, ou bien

b) on imprègne avec des sels, des nitrates de préférence, des éléments promoteurs, le mélange d'oxydes obtenu par dissociation thermique d'une solution de carbonates aminés de cuivre et de zinc en présence d'oxyde d'aluminium en suspension;

on procède ensuite à une calcination du produit obtenu selon la procédure (a) ou (b) à une température de 350 à 450°C, de préférence de 380 à 400°C.

5. Catalyseur selon la revendication 4, caractérisé par le fait que l'on obtient le précipité selon la variante (a) par précipitation continue avec du carbonate alcalin, des éléments constitutifs principaux et des éléments promoteurs solubles dans l'eau.

6 Catalyseur selon la revendication 5, caractérisé par le fait que l'on introduit dans une chambre de mélange munie d'un dispositif agitateur une solution aqueuse des éléments constitutifs principaux et promoteurs solubles dans l'eau, ainsi qu'une solution aqueuse de carbonate alcalin, et que l'on transfère la suspension de précipité qui se forme dans la chambre de mélange dans un réservoir de calmage, pour maturation.

7. Catalyseur selon l'une des revendications de 1 à 6, caractérisé par le fait que l'on soumet les catalyseurs de départ imprégnés avec les composés promoteurs à une réimprégnation avec un composé de potassium et à une recalcination, la teneur en potassium (calculée en poids de $K_2O$) étant de 0,03 à 3,4 pour cent, de préférence de 1,7 à 2,5 pour cent du poids.

8. Catalyseur selon la revendication 7, caractérisé par le fait que comme composés du potassium on utilise de l'hydroxyde de potassium, du carbonate de potassium, de l'hydrocarbonate de potassium, de l'acétate de potassium, du chromate ou bichromate de potassium ou des mélanges de ces différents composés, et que la recalcination s'effectue à une température de 350 à 450°C, de préférence de 380 à 400°C.

9. Catalyseur selon l'une des revendication de 1 à 8, caractérisé par le fait que pour activation on le soumet à un traitement réducteur complémentaire.

10. Catalyseur selon la revendication 9, caractérisé par le fait que ce traitement réducteur complémentaire se fait tout d'abord avec un gaz inerte, de l'azote par exemple, contenant une petite quantité d'hydrogène et que l'on augmente progressivement la teneur en hydrogène en même temps que l'on élève la température de 170 à 350°C.

11. Procédé de synthèse de mélanges d'alcools contenant du méthanol et des alcools supérieurs, caractérisé en ce que le catalyseur utilisé est le catalyseur décrit dans les revendications 1 à 10.

12. Procédé de synthèse selon la revendication 11, caractérisé par le fait que la synthèse s'effectue à une température de 250 à 400°C, de préférence à 350°C, avec une pression de 80 à 150 bars, de préférence de 100 bars et une vitesse volumétrique de 1000 à 10000, de préférence de 3000 litres par heure et par litre de catalyseur, avec un gaz de processus contenant, en volume, 25 à 30 pour cent, de préférence 27 pour cent de CO, 0 à 8 pour cent de $N_2$, 0 à 5 pour cent de $CO_2$, 0 à 5 pour cent de $CH_4$, le reste étant de l'hydrogène, $H_2$.